(19) Europäisches Patentamt
European Patent Office
Office européen des brevets

(11) **EP 1 687 045 B1**

(12) **EUROPÄISCHE PATENTSCHRIFT**

(45) Veröffentlichungstag und Bekanntmachung des Hinweises auf die Patenterteilung:
**23.12.2009 Patentblatt 2009/52**

(21) Anmeldenummer: **04765103.9**

(22) Anmeldetag: **11.09.2004**

(51) Int Cl.:
**A61M 1/36** (2006.01)

(86) Internationale Anmeldenummer:
**PCT/EP2004/010180**

(87) Internationale Veröffentlichungsnummer:
**WO 2005/058390 (30.06.2005 Gazette 2005/26)**

(54) **VORRICHTUNG ZUM ERKENNEN VON STÖRUNGEN DES BLUTFLUSSES IN EINEM EXTRAKORPORALEN BLUTKREISLAUF**

DEVICE FOR IDENTIFYING IMPAIRMENTS OF THE BLOOD FLOW IN AN EXTRA-CORPOREAL BLOOD CIRCUIT

DISPOSITIF POUR DETECTER DES TROUBLES DE LA CIRCULATION SANGUINE DANS UN CIRCUIT DE CIRCULATION SANGUINE EXTRACORPOREL

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.11.2003 DE 10355042**

(43) Veröffentlichungstag der Anmeldung:
**09.08.2006 Patentblatt 2006/32**

(73) Patentinhaber: **Fresenius Medical Care Deutschland GmbH**
**61352 Bad Homburg v.d.H. (DE)**

(72) Erfinder:
• **KOPPERSCHMIDT, Pascal**
**97456 Dittelbrunn (DE)**
• **GROSS, Malte**
**97464 Niederwerm (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**EP-A- 0 330 761     EP-A- 1 245 242**

**Beschreibung**

Die Erfindung betrifft eine Vorrichtung zum Erkennen von Störungen des Blutflusses in einem extrakorporalen Blutkreislauf während einer extrakorporalen Blutbehandlung mit einer extrakorporalen Blutbehandlungsvorrichtung.

Zur Entfernung von harnpflichtigen Substanzen und zum Flüssigkeitsentzug werden beim akuten und chronischen Nierenversagen verschiedene Verfahren zur apparativen Blutbehandlung eingesetzt. Bei der Hämodialyse (HD) wird das Blut eines Patienten in einem extrakorporalen Blutkreislauf durch eine Kammer eines von einer semipermeablen Membran in zwei Kammern unterteilten Dialysators geleitet, während die andere Kammer von einer Dialysierflüssigkeit durchströmt wird. Über die Membran des Dialysators findet sowohl ein konvektiver als auch diffusiver Stoffaustausch statt. Bei der Hämofiltration (HF) liegt nur ein konvektiver Stoffaustausch vor. Eine Kombination aus beiden Verfahren ist die Hämodiafiltration (HDF).

Die bekannten Vorrichtungen zur Hämodiafiltration verfügen über eine oder mehrere Substitutionspumpen, mit denen physiologische Flüssigkeit dem Blut des Patienten zugeführt wird, während über den Dialysator bzw. Filter der Hämodiafiltrationsvorrichtung Flüssigkeit entzogen wird. Die physiologische Flüssigkeit kann stromauf bzw. stromab des Dialysators dem arteriellen bzw. venösen Zweig des extrakorporalen Kreislaufs zugeführt werden. Die Substitution der Flüssigkeit vor Eintritt des Blutes in den Dialysator bzw. Filter wird als Prädilution und die Substitution nach Austritt des Blutes aus dem Dialysator bzw. Filter als Postdilution bezeichnet.

Es hat sich gezeigt, dass eine HDF-Blutbehandlung, bei der eine Postdilution erfolgt, bei gleicher Substitutionsfördermenge gegenüber einer Behandlung, bei der eine Prädilution erfolgt, eine höhere Effizienz hat. Die höhere Reinigungsleistung bei postdilutiver Zugabe von Substitutionsflüssigkeit gegenüber prädilutiver Substitution ist darauf zurückzuführen, dass bei Postdilution das Filtrat in vollem Umfang aus dem zu reinigenden Blut gewonnen wird, während bei Prädilution das mit Substitutionsflüssigkeit verdünnte Blut in den Dialysator bzw. Filter strömt, bevor uränische Giftstoffe während des Durchströmens durch den Patienten aufgenommen werden können.

Ein Nachteil der Prädilution liegt darin, dass eine zu hohe Ultrafiltrationsrate, d.h. ein zu großer Flüssigkeitsentzug über die Membran des Dialysators bzw. Filters zu einer Eindickung des Blutes und zur Erhöhung des Strömungswiderstandes im Dialysator bzw. Filter führt.

Es hat sich gezeigt, dass die Blutbehandlungseinrichtungen bei erhöhtem Strömungswiderstand nicht mehr in der Lage sind, das zu reinigende Blut mit der eingestellten Förderrate zu fördern, wodurch die Effektivität der Blutbehandlung verringert wird. Der Strömungswiderstand im Dialysator bzw. Filter kann aber auch einen vollständigen Verschluß der Membran zur Folge haben. Dann ist die Behandlung unterbrochen, wobei gegebenenfalls das gesamte Blutschlauchsystem zu ersetzen ist.

Der blutseitige Strömungswiderstand im Dialysator bzw. Filter ist vom Hämatokrit des Blutes, den Eigenschaften der Membran des Dialysators bzw. Filters und dem Verhältnis der Fördermengen von Blutpumpe und Substitutionspumpe abhängig. Ein Anstieg des Strömungswiderstandes führt zu einem Druckanstieg zwischen der stromauf des Dialysators bzw. Filters angeordneten Blutpumpe und dem Dialysator bzw. Filter. Da sich sowohl der Hämatokrit des Blutes als auch die Membraneigenschaften im Laufe der Behandlung ändern, wird eine den Bedingungen angepaßte Substitution zur Erhöhung der Behandlungseffizienz angestrebt.

Bereits existierende Regelungen zur Substituatförderung basieren auf einem festen Verhältnis der Förderraten von Blutpumpe und Substituatpumpe. Aus der EP 1 175 917 A1 ist eine Hämodialysevorrichtung bekannt, bei der die Regelung zweier Substituatpumpen bei prä- und/oder postdiluter Substitution auf der Grundlage der Veränderung des Transmembrandrucks oder des Hämatokrit erfolgt. Zur Ermittlung des Transmembrandrucks schlägt die EP 1 175 917 A1 vor, den Druck sowohl im extrakorporalen Blutkreislauf als auch im Dialysierflüssigkeitssystem zu messen.

Die DE 38 06 248 A1 beschreibt ein Schutzsystem zur Drucküberwachung des Flüssigkeitskreislaufs eines medizinischen Gerätes, bei dem nicht nur der statische Druck, sondern auch im Flüssigkeitskreislauf vorhandene Druckschwankungen ausgewertet werden. Die DE 38 06 248 A1 schlägt vor, zur Detektion von Unterbrechungen der Strömung in dem Flüssigkeitskreislauf die Phasenverschiebung von Druckpulsen zu erfassen, die mit einem Drucksensor detektiert werden.

Aus der US 2002/0174721 A1 ist ein Verfahren zum Erkennen von Stenosen in einem Schlauchleitungssystem während einer extrakorporalen Blutbehandlung bekannt. Zur Erkennung einer Stenose wird das Frequenzspektrum eines oszillierenden Drucksignals analysiert, das sich im extrakorporalen Blutkreislauf fortpflanzt. Auf eine Stenose wird darin geschlossen, wenn sich die Dämpfung mindestens einer Oberschwingung des oszillierenden Drucksignals ändert.

Der Erfindung liegt die Aufgabe zugrunde, eine zuverlässig arbeitende Vorrichtung bereitzustellen, die eine Erkennung von Störungen des Blutflusses in einem extrakorporalen Blutkreislauf insbesondere die Erhöhung des Strömungswiderstandes bis zum möglichen Verschluss der Membran des Dialysators oder Filters, erlaubt. Insbesondere ist eine Aufgabe der Erfindung, eine Vorrichtung anzugeben, mit der Störungen des Blutflusses schon frühzeitig erkannt werden können. Die Lösung dieser Aufgabe erfolgt erfindungsgemäß mit den im Patentanspruch 1 angegebenen Merkmalen.

Die erfindungsgemäße Vorrichtung beruht auf der Analyse eines sich im extrakorporalen Blutkreislauf fortpflanzenden oszillierenden Drucksignals. Bei der Vorrichtung wird nicht die Grundschwingung des oszillierenden Drucksignals, sondern mindestens einer der harmonischen Anteile des Drucksignals analysiert. Die Analyse des Phasenwinkels mindestens einer Oberschwingung des Drucksignals erlaubt die Detektion einer sich anbahnenden Blutverdickung, so dass rechtzeitig Gegenmaßnahmen eingeleitet werden können. Auf eine Störung des Blutflusses wird dann geschlossen, wenn der Phasenwinkel mindestens einer Oberschwingung eine charakteristische Veränderung erfährt. Prinzipiell ist es möglich, auf eine Störung des Blutflusses dann zu schließen, wenn eine charakteristische Veränderung des Phasenwinkels nur einer Oberschwingung vorliegt. Zur Erhöhung der Sicherheit können aber auch die Phasenwinkel mehrerer Oberschwingungen analysiert werden, wobei die Auswertung mit den bekannten statistischen Verfahren erfolgen kann.

Ein Vorteil der erfindungsgemäßen Vorrichtung liegt darin, dass das oszillierende Drucksignal nur an einer Stelle des extrakorporalen Blutkreislaufs gemessen zu werden braucht. Daraus ergibt sich ein relativ einfacher apparativer Aufbau. Vorzugsweise wird der Druck im venösen Zweig des extrakorporalen Kreislaufs stromab der Blutbehandlungseinheit gemessen, d.h. nachdem Flüssigkeit dem Blut entzogen worden ist. Die Messung kann mit einem venösen Drucksensor erfolgen, der ohnehin in den bekannten Blutbehandlungsvorrichtungen vorhanden ist. Prinzipiell ist auch eine Druckmessung in der Blutbehandlungseinheit, beispielsweise in der Filterkappe oder den Hohlfasern des Dialysators möglich, wobei aufgrund von Reflektionen eine Veränderung des Phasenwinkels grundsätzlich auch stromauf der Blutbehandlungseinheit nachweisbar sind. In der Praxis wird aber davon abzusehen sein.

Die erfindungsgemäße Vorrichtung macht weitere Messzellen, beispielsweise zur Bestimmung des Transmembrandrucks oder des Hämatokrits nicht erforderlich. Auch ist zusätzliche Hardware oder Software zur Bestimmung beispielsweise der Filterkoeffizienten nicht notwendig. Ferner kann das Blutschlauchsystem der Blutbehandlungsvorrichtung unverändert bleiben.

Als Alarmkriterium kann anstelle einer Differentialbetrachtung auch eine kritische absolute oder relative Veränderung des Absolutwertes der Phase im Vergleich zu einem Grenzwert ausgewertet werden.

Zur Analyse einer oder mehrerer Oberschwingungen des oszillierenden Drucksignals wird vorzugsweise die Änderung des Phasenwinkels zumindest einer Oberschwingung in einer vorgegebenen Zeiteinheit mit einem vorgegebenen Grenzwert verglichen, wobei eine Störung detektiert wird, wenn der Betrag der Änderung des Phasenwinkels größer als der vorgegebene Grenzwert ist. In Versuchen hat sich gezeigt, dass der Phasenwinkel einer Oberschwingung vor dem Auftreten eines starken Anstiegs des Strömungswiderstandes plötzlich relativ stark abfällt. Es hat sich gezeigt, dass eine charakteristische Veränderung des Phasenwinkels besonders deutlich bei Oberschwingungen höherer Ordnung auftritt. Die charakteristische Veränderung wird dabei um so deutlicher, je größer die Ordnungszahl der Oberschwingung ist.

Bei Störungen des Blutflusses wird vorzugsweise ein Eingriff in die Steuerung der extrakorporalen Blutbehandlungsvorrichtung vorgenommen, um Gegenmaßnahmen automatisch einleiten zu können. Grundsätzlich ist es aber auch möglich, nur die Störung des Blutflusses zu detektieren, so dass Gegenmaßnahmen gegebenenfalls manuell eingeleitet werden können.

Besondere Vorteile bietet die erfindungsgemäße Vorrichtung bei der Hämodialyse- und/oder Hämofiltration. Als Eingriff in die Steuerung der Hämodialyse- und/oder Hämofiltrationsvorrichtung kann bei einer Störung eine bestimmte Menge an Substitutionsflüssigkeit in einem vorgegebenen Zeitintervall dem Blut stromauf des Dialysators zugeführt werden. Dadurch wird erreicht, dass sich das Blut nicht verdickt. Alternativ kann aber auch die Ultrafiltrationsrate in einem vorgegebenen Zeitintervall verringert werden. Auch dadurch wird erreicht, dass sich das Blut nicht verdickt. Es ist aber auch möglich, sowohl die Förderrate der Substitutionsflüssigkeit zu erhöhen als auch die Ultrafiltrationsrate zu verringern.

Zur Ermittlung des Phasenwinkels mindestens einer Oberschwingung wird vorteilhafterweise eine Fourier-Analyse des oszillierenden Drucksignals durchgeführt. Dies kann mit dem bekannten Fourier-Analyse-Einrichtungen erfolgen, die nach den bekannten Algorithmen arbeiten.

Die Fourier-Analyse muss nicht in Form einer auf Software basierenden mathematischen Auswertung erfolgen. Da die zu filternden Frequenzen durch die Pumpendrehzahl bekannt sind, können auch Hardware-Filter wie Bandpass- oder Kammfilter Verwendung finden, die mit diskreten Bauelementen realisiert werden. Dies kann insbesondere dann vorteilhaft sein, wenn die zur Verfügung stehende Rechenkapazität begrenzt ist und die Hardwarekomponenten preiswert und platzsparend zur Verfügung stehen.

Für die erfindungsgemäße Vorrichtung ist grundsätzlich unerheblich, wie das oszillierenden Drucksignal generiert wird. Vorzugsweise werden die Druckpulse der Blutpumpe, insbesondere einer okkludierenden Blutpumpe, beispielsweise einer Rollenpumpe gemessen, mit der das Blut im arteriellen Zweig gefördert wird.

Im Folgenden wird der mathematische Zusammenhang zur Berechnung der Winkelgeschwindigkeit im Einzelnen erläutert.

Als Messgröße dient der venenseitig ermittelte mit $\omega$ oszillierende Druck $p_{ven}(t)$, welcher mittels der Fourieranalyse, der Fast Fourier Analyse (FFT) oder anderen geeigneten Filterverfahren (z.B. Kammfilter, Bandpassfilter, etc.) in höhere spektrale Harmonische der Ordnung n zerlegt wird.

$$[1] \quad A_n = f_n(\omega, p_{ven})$$

$$[2] \quad B_n = f^*_n(\omega, p_{ven})$$

Beispielsweise kann $f$ und $f^*$ als komplex konjugierte Funktion von $f$, die folgende Form besitzen:

$$[3] \quad f_n(\omega, p_{ven}) = \int_{T=\frac{2\pi}{\omega}} p_{ven}(t)\,\sin(n\omega t)\,dt, \quad (n \in \mathrm{N})$$

$$[4] \quad f^*_n(\omega, p_{ven}) = \int_{T=\frac{2\pi}{\omega}} p_{ven}(t)\,\cos(n\omega t)\,dt, \quad (n \in \mathrm{N})$$

Die reellen Koeffizienten $A_n$ und $B_n$ der Zerlegung bilden eine komplexe Zahl $Z_n$ der Form:

$$[5] \quad Z_n = A_n + iB_n \qquad (n \in \mathrm{N}),$$

wobei $Z_n$ ein Vektor der komplexen Zahlenebene darstellt. Der Betrag des Vektors und der Winkel $\phi$ des Vektors in Polarkoordinaten lassen sich ermitteln nach:

$$[6] \quad \text{Betrag: } |Z_n| = |A_n + iB_n| = \sqrt{(A_n + iB_n)(A_n - iB_n)} = \sqrt{A_n^2 + B_n^2}$$

$$(n \in \mathrm{N})$$

$$[7] \quad \text{Phase: } \phi_n = \arctan\frac{A_n}{B_n} \qquad (n \in \mathrm{N})$$

Der zeitliche Verlauf, der in [7] ermittelten Phase wird beobachtet und gegenüber einer kritischen Geschwindigkeit $\omega_{crit}$ bewertet:

$$[8] \quad \frac{d}{dt}\phi_n - \omega_{crit,n} = \pm\Delta_n$$

Die Bewertung $\Delta_n$ ist ein Maß für die Änderung des blutseitigen Strömungsverhaltens durch den Dialysator während einer Dialysebehandlung mit ausgeprägtem konvektiven Stofftransport (HDF, HF), insbesondere bei großer Ordnung n.
Nachfolgend wird die erfindungsgemäße Vorrichtung unter Bezugnahme auf die Figuren näher erläutert.
Es zeigen:

Fig. 1     eine Hämodialysevorrichtung zusammen mit einer Vorrichtung zum Er- kennen von Störungen des Blutflusses in vereinfachter schematischer Dar- stellung,

Fig. 2 die Veränderung der normierten Phasenwinkel von Oberschwingungen des oszillierenden Drucksignals in Abhängigkeit von der Behandlungszeit bei sukzessiver Erhöhung der Förderrate der Substitutionsflüssigkeit und

Fig. 3 den Blutdruck im arteriellen Zweig des extrakorporalen Blutkreislaufs als Funktion der Behandlungszeit bei sukzessiver Erhöhung der Förderrate der Substitutionsflüssigkeit.

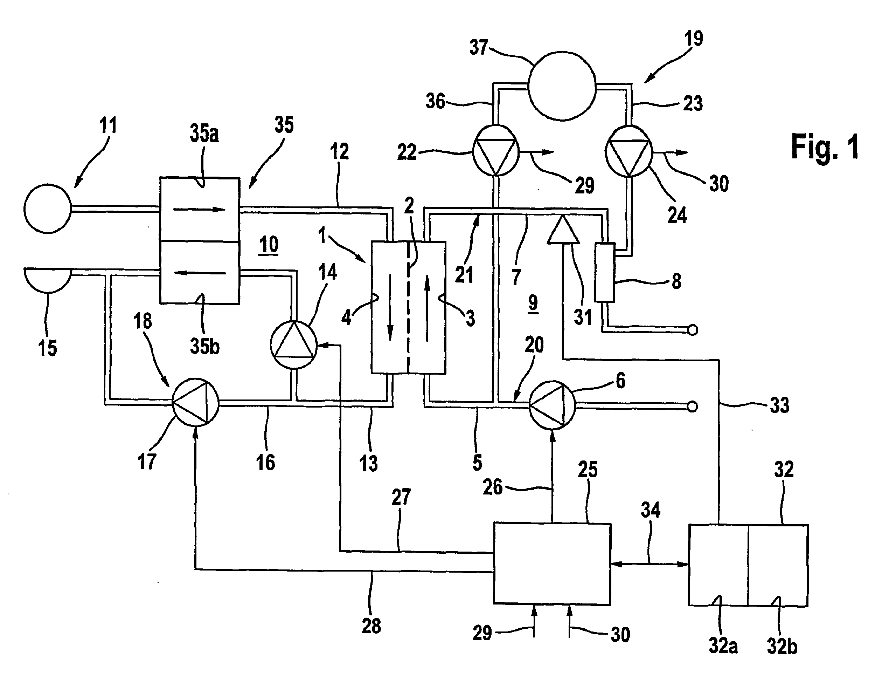

Fig. 1 zeigt die wesentlichen Komponenten einer Hämodialysevorrichtung in vereinfachter schematischer Darstellung. Die Hämodialysevorrichtung weist einen Dialysator 1 auf, der durch eine semipermeable Membran 2 in eine Blutkammer 3 und eine Dialysierflüssigkeitskammer 4 getrennt ist. Der Einlass der Blutkammer ist mit einem Ende der Blutzuführleitung 5 verbunden, in die eine Blutpumpe 6 geschaltet ist, während der Auslass der Blutkammer 3 mit einem Ende einer Blutabführleitung 7 verbunden ist, in die eine Tropfkammer 8 geschaltet ist. Blutzuführ- und -abführleitung 5, 7 bilden mit der Blutkammer 3 des Dialysators 1 den extrakorporalen Blutkreislauf 9 der Dialysevorrichtung. Bei der Blutzuführ- und -abführleitung 5, 7 handelt es sich um Schlauchleitungen eines in die Dialysevorrichtung eingelegten Schlauchsets.

Das Dialysierflüssigkeitssystem 10 der Dialysevorrichtung umfasst eine Einrichtung 11 zur Bereitstellung von Dialysierflüssigkeit, die über den ersten Abschnitt einer Dialysierflüssigkeitzuführleitung 12 mit dem Einlass der ersten Kammerhälfte 35a einer Bilanziereinrichtung 35 verbunden ist. Der zweite Abschnitt der Dialysierflüssigkeitszuführleitung 12 verbindet den Auslass der ersten Bilanzierkammerhälfte 35a mit dem Einlass der Dialysierflüssigkeitskammer 4. Der Auslass der Dialysierflüssigkeitskammer 4 ist über den ersten Abschnitt einer Dialysierflüssigkeitsabführleitung 13 mit dem Einlass der zweiten Bilanzierkammerhälfte 35b verbunden. In den ersten Abschnitt der Dialysierflüssigkeitsabführleitung 13 ist eine Dialysierflüssigkeitspumpe 14 geschaltet. Der Auslass der zweiten Bilanzierkammerhälfte 35b ist über den zweiten Abschnitt der Dialysierflüssigkeitsabführleitung 13 mit einem Auslauf 15 verbunden. Stromauf der Dialysierflüssigkeitspumpe 14 zweigt von der Dialysierflüssigkeitsabführleitung 13 eine Ultrafiltratleitung 16 ab, die ebenfalls zu dem Auslauf 15 führt. In die Ultrafiltratleitung 16 ist eine Ultrafiltrationspumpe 17 geschaltet. Die Bilanziereinrichtung mit nur einer Bilanzkammer, die zwei Bilanzkammerhälften aufweist, dient nur der Erläuterung. Anstelle von einer Bilanzkammer können auch zwei Bilanzkammern vorgesehen sein. Anstelle einer volumetrischen Bilanziereinrichtung können auch gravimetrische Wägemittel vorgesehen sein.

Während der Dialysebehandlung wird die Blutkammer 3 von dem Blut des Patienten und die Dialysierflüssigkeitskammer 4 des Dialysators 1 von der Dialysierflüssigkeit durchströmt. Die Bilanziereinrichtung 35 stellt sicher, dass nur soviel Dialysierflüssigkeit über die Dialysierflüssigkeitszuführleitung 12 zufließen kann, wie Dialysierflüssigkeit über die Dialysierflüssigkeitsabführleitung 13 abfließen kann. Mit der Ultrafiltrationspumpe 17 kann dem Patienten Flüssigkeit entzogen werden. Die Ultrafiltrationspumpe 17 ist somit Teil einer Einrichtung zum Entziehen von Flüssigkeit aus dem Blut, die als Ultrafiltrationseinrichtung 18 bezeichnet wird.

Um dem Patienten Flüssigkeit wieder zuzuführen, verfügt die Dialysevorrichtung über eine Substitutionseinrichtung 19, mit der eine Substitutionsflüssigkeit (Substituat) dem Blut zugeführt werden kann, dass durch den arteriellen Zweig 20 (Prädilution) und/oder den venösen Zweig 21 (Postdilution) des extrakorporalen Blutkreislaufs 9 strömt. Die Substitutionseinrichtung 19 weist eine Einrichtung 20 zur Bereitstellung von Substituat auf, von der eine erste Substituatleitung 36, in die eine erste Substituatpumpe 22 geschaltet ist, zu dem Abschnitt der Blutzuführleitung 5 zwischen Blutpumpe 6 und Blutkammer 3 führt. Eine zweite Substituatleitung 23, in die eine zweite Substitutatpumpe 24 geschaltet ist, führt von der Einrichtung 37 zur Bereitstellung von Substituat zu der Tropfkammer 8.

Darüber hinaus weist die Dialysevorrichtung eine zentrale Steuereinheit 25 auf, die über Steuerleitungen 26-30 mit der Blutpumpe 6, der Dialysierflüssigkeitspumpe 14, der Ultrafiltrationspumpe 17 sowie der ersten und zweiten Substitutionspumpe 22, 24 verbunden ist.

Die erfindungsgemäße Vorrichtung zum Erkennen von Störungen des Blutflusses wird als Bestandteil der Blutbehandlungsvorrichtung beschrieben, da die Blutbehandlungsvorrichtung bereits über die erforderliche Hardware verfügt. Die erfindungsgemäße Vorrichtung kann grundsätzlich aber auch eine separate Einheit bilden.

Die Vorrichtung zum Erkennen von Störungen weist einen im venösen Zweig 21 des extrakorporalen Kreislaufs 9 stromauf der Tropfkammer 8 angeordneten venösen Drucksensor 31 und eine Auswerteinheit 32 auf, die das Ausgangssignal des Drucksensors über eine Signalleitung 33 empfängt. Die Auswerteinheit 32 ist über eine Datenleitung 34 mit der zentralen Steuereinheit 25 der Dialysevorrichtung verbunden. Auswerteinheit 32 und Steuereinheit 25 tauschen die für die Bluthandlung erforderlichen Daten untereinander aus, so dass die Steuereinheit einen Eingriff in die Maschinensteuerung dann vornehmen kann, wenn die Auswerteinheit einen Störfall detektiert.

Im Folgenden wird die Funktion der Auswerteinheit 32 im Einzelnen beschrieben.

Die Auswerteinheit 32 verfügt über eine Fourier-Analyse-Einrichtung 32a, die das Ausgangssignal 33 des venösen Drucksensors 31 analysiert. Die Rollenpumpe 6 erzeugt oszillierende Druckpulse, die sich über den arteriellen und venösen Zweig 20, 21 des extrakorporalen Kreislauf 9 fortpflanzen. Die oszillierenden Druckpulse werden mit dem venösen Drucksensor 31 gemessen und mit der Fourier-Analyse-Einrichtung 32a der Auswerteinheit 32 analysiert.

Das oszillierende Drucksignal weist einen statischen Anteil ($\omega$) = 0) und harmonische Anteile auf. Da es sich bei der Rollenpumpe um eine Pumpe mit zwei Rollen handelt, sind die ungeraden Oberschwingungen ($1\omega$, $3\omega$, $5\omega$ ...) zu vernachlässigen. Die Fourier-Analyse-Einrichtung 32a zerlegt das oszillierende Drucksignal in einen statischen Anteil und die geraden Oberschwingungen ($2\omega$, $4\omega$, $6\omega$ ...), wobei jeweils der Phasenwinkel der Oberschwingungen ermittelt wird.

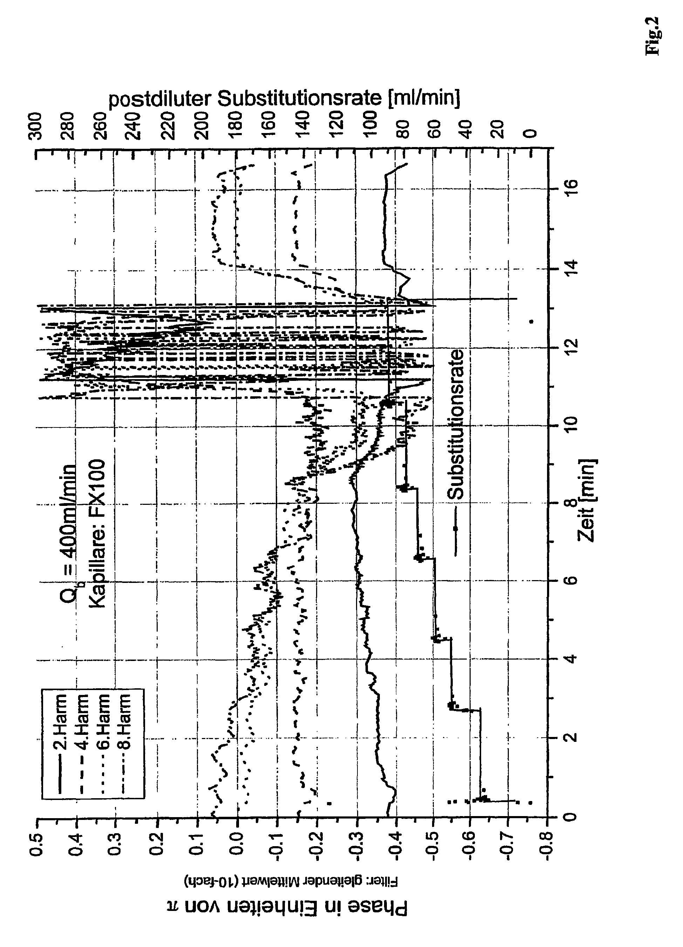

Figur 2 zeigt den Phasenwinkel der 2., 4., 6. und 8. Oberschwingung des oszillierenden Drucksignals als Funktion der Behandlungszeit bei einer in-vitro HDF-Behandlung mit Postdilution. Die Substituatförderrate wurde sukzessiv auf 90 ml/min gesteigert, bis der Druck im Blutschlauchsegment zwischen Blutpumpe und Dialysator instabil wurde. Gleichzeitig wurde die Ultrafiltrationsrate in gleichem Maße erhöht. Es hat sich gezeigt, dass eine sich anbahnende Blutverdickung durch einen schnellen und deutlich abfallenden Phasenwinkel der einzelnen Oberschwingungen zu erkennen ist. Die Postdilution und die Erhöhung der Ultrafiltrationsrate hat zur Folge, dass die Hohlfasern des Dialysators verstopfen. Die erhöhte Substitutionsrate als Folge der Erhöhung der Ultrafiltrationsrate ist dagegen nicht die primär beeinflussende Größe. Der Phasenwinkel der Oberschwingungen fällt bei ca. 8-10 Minuten stark ab. Besonders stark fällt der Phasenwinkel bei den Oberschwingungen höherer Ordnung ab. Bei weiterer Erhöhung der Substitutionsrate kommt es bei 10-13 Minuten zu nahezu chaotischen Fluktuationen des Phasenwinkels aufgrund der sich bildenden Verstopfung der Membran des Dialysators.

Neben der Fourier-Analyse-Einrichtung 32a weist die Auswerteinheit 32 eine Recheneinheit 32b zum Detektieren einer charakteristischen Veränderung des Phasenwinkels einzelner Oberschwingungen auf. Im Folgenden wird nur die Analyse einer Oberschwingung, d.h. der 8. Oberschwingung beschrieben. Die Auswertung kann aber auch auf der Grundlage mehrerer Oberschwingungen erfolgen.

Die Recheneinheit 32b weist einen Differentiator auf, der das Phasenwinkel-Signal differenziert. Das Differential des Phasen-Signals als Funktion der Zeit ist ein Maß für den Abfall des Phasenwinkels. Die Recheneinheit vergleicht das Differential des Phasenwinkel-Signals mit einem vorgegebenen Grenzwert. Wenn das Differential den Grenzwert übersteigt, wird ein Störfall angenommen. Es kann ein akustischer und/oder optischer Alarm gegeben werden. Da die Detektion des Störfalls erfolgt, bevor die Membran des Dialysators verstopft ist, können noch rechtzeitig Gegenmaßnahmen eingeleitet werden.

Die Auswerteinheit 32 sendet im Falle der Detektion eines Störfalls über die Datenleitung 34 ein Signal an die zentrale Steuereinheit 25 der Dialysevorrichtung zur Einleitung eines Eingriffs in die Maschinensteuerung. Die zentrale Steuereinheit 25 steuert die erste Substituatpumpe 22 derart an, dass einer Verdickung des Blutes entgegengewirkt wird. Hierzu wird die Förderrate der Substituatpumpe 22 für ein vorgegebenes Zeitintervall erhöht, um eine bestimmte Menge an Substitutionsflüssigkeit stromauf der Blutkammer 3 des Dialysators 1 zuzuführen, so dass das in den Dialysator strömende Blut verdünnt wird. Die Steuereinheit 25 kann aber auch die Ultrafiltrationseinrichtung 18 derart ansteuern, dass die Ultrafiltrationsrate für ein vorgegebenes Zeitintervall verringert wird, wodurch der Verdickung des Blutes entgegengewirkt wird. Beide Gegenmaßnahmen können aber auch gleichzeitig eingeleitet werden.

Als Gegenmaßnahme kann auch eine Regelung dergestalt vorgesehen sein, dass die Rückführung der Phase auf einen Anfangswert als Zielwert angestrebt wird. Dies kann insbesondere durch die Zuführung der Postdilutionsflüssigkeit als Prädilutionsflüssigkeit erreicht werden. Wenn beispielsweise am Anfang eine Postdilution erfolgt, kann bei einer Veränderung des Phasenwinkels die Förderrate der ersten Substituatpumpe 22 für Prädilution erhöht werden, während die Förderrate der zweiten Substituatpumpe 23 für Postdilution verringert oder konstant gehalten wird. Je nach Abweichung kann dann eine Gegenregelung nach den bekannten Verfahren beispielsweise mit einem P-, PI-, oder PID-Regler erfolgen.

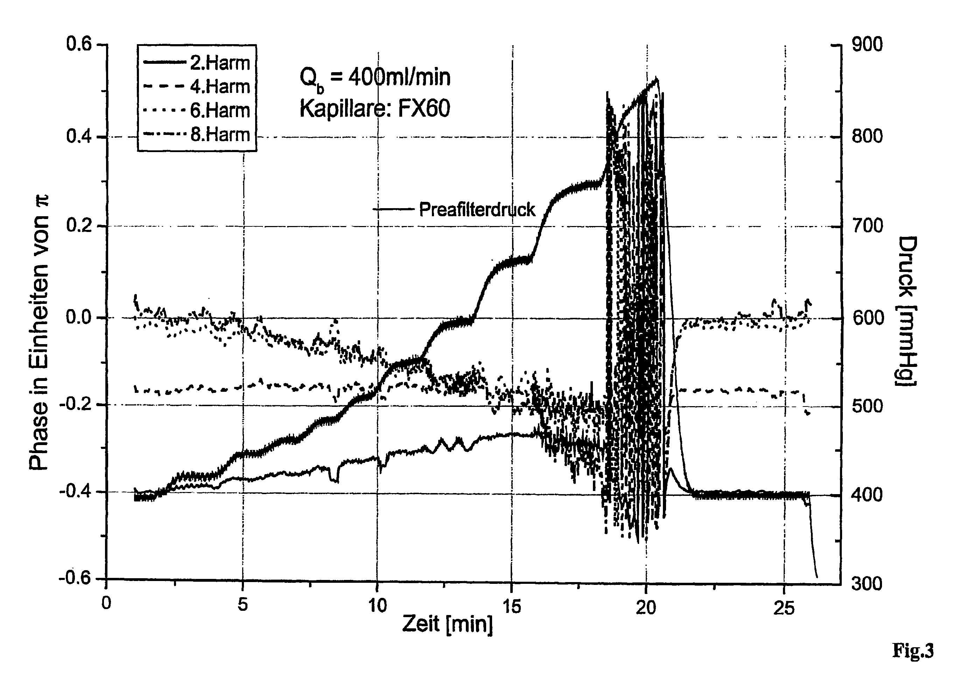

Figur 3 zeigt den arteriellen Druck im Blutschlauchsegment stromauf der Blutkammer 3 zwischen Blutpumpe 6 und Blutkammer 3 des Dialysators 1 (Präfilterdruck) als Funktion der Zeit bei sukzessiver Erhöhung der Substitutionsrate und der Ultrafiltrationsrate und Postdilution. Gleichzeitig sind die Phasen der 2., 4., 6., und 8. höheren Harmonischen des oszillierenden Drucks wiedergegeben. Es ist zu erkennen, dass nach jeder Erhöhung der Substituatrate sich im stabilen Fall nach kurzer Zeit ein fester Präfilterdruck einstellt. Kommt es aufgrund eines ungünstigen Verhältnisses zwischen den Fördermengen der Blutpumpe und der Substituatpumpe zur Instabilität, so steigt der Präfilterdruck zeitlich monoton an, ohne einen konstanten Wert anzunehmen. Dieser Anstieg koinzidiert mit einer drastischen Zunahme des Phasenwinkels. Dabei könnte der Druckanstieg sogar zu einer Ruptur der Dialysatormembran führen.

**Patentansprüche**

1. Vorrichtung zum Erkennen von Störungen des Blutflusses in einem extrakorporalen Blutkreislauf während einer extrakorporalen Blutbehandlung mit einer extrakorporalen Blutbehandlungsvorrichtung, die einen extrakorporalen Blutkreislauf (9) mit einem zu einer Blutbehandlungseinheit (1) führenden arteriellen Zweig (20) und einem von der Blutbehandlungseinheit abgehenden venösen Zweig (21) aufweist, wobei die Vorrichtung zum Erkennen von Störungen des Blutflusses aufweist:

Mittel (6) zum Generieren eines sich im extrakorporalen Blutkreislauf fortpflanzenden oszillierenden Drucksignals,

Mittel (31) zum Messen des oszillierenden Drucksignals und

Mittel (32) zum Analysieren des oszillierenden Drucksignals, **dadurch gekennzeichnet,**

**dass** die Mittel (32) zum Analysieren des oszillierenden Drucksignals Mittel (32a) zum Bestimmen des Phasenwinkels mindestens einer Oberschwingung des oszillierenden Drucksignals und Mittel (32b) zum Detektieren einer Veränderung des Phasenwinkels aufweisen.

2. Vorrichtung nach Anspruch 1, **dadurch gekennzeichnet, dass** die Mittel (32b) zum Detektieren einer Veränderung des Phasenwinkels derart ausgebildet sind, dass die Änderung des Phasenwinkels mindestens einer Oberschwingung in einem vorgegebenen Zeitintervall ermittelt und die zeitliche Änderung des Phasenwinkels der Oberschwingung mit einem vorgegebenen Grenzwert verglichen wird, wobei eine Störung detektiert wird, wenn der Betrag der Änderung des Phasenwinkels grösser als der vorgegebene Grenzwert ist.

3. Vorrichtung nach Anspruch 1 oder 2, **dadurch gekennzeichnet, dass** die Vorrichtung zum Erkennen von Störungen des Blutflusses ferner Mittel (25, 32) zum Vornehmen eines Eingriffs in die Steuerung der extrakorporalen Blutbehandlungsvorrichtung aufweist.

4. Vorrichtung nach einem der Ansprüche 1-3, **dadurch gekennzeichnet, dass** die extrakorporalen Blutbehandlungsvorrichtung eine Hämodialyse- und/oder Hämofiltrationsvorrichtung und die Blutbehandlungseinheit (1) ein Dialysator bzw. ein Filter ist, wobei die extrakorporale Blutbehandlungsvorrichtung aufweist:

eine Ultrafiltrationseinrichtung (18), die dem durch den extrakorporalen Blutkreislauf strömenden Blut mit einer vorgegebenen Ultrafiltrationsrate Flüssigkeit entzieht und eine Substitutionseinrichtung (19), die Substitutionsflüssigkeit dem durch den extrakorporalen Blutkreislauf strömenden Blut stromauf und/oder stromab des Dialysators (1) bzw. Filters zuführt.

5. Vorrichtung nach Anspruch 4, **dadurch gekennzeichnet, dass** die Mittel (25, 32) zum Vornehmen eines Eingriffs in die Steuerung der Hämodialyse- und/oder Hämofiltrationsvorrichtung die Ultrafiltrationseinrichtung (18) bei der Detektion einer Störung derart ansteuert, dass die Ultrafiltrationsrate in einem vorgegebenen Zeitintervall verringert wird.

6. Vorrichtung nach Anspruch 4, **dadurch gekennzeichnet, dass** die Mittel (25, 32) zum Vornehmen eines Eingriffs in die Steuerung der Hämodialyse- und/oder Hämofiltrationsvorrichtung die Substitutionseinrichtung (19) bei der Detektion einer Störung derart ansteuert, dass stromauf des Dialysators (1) bzw. Filters eine bestimmte Menge an Substitutionsflüssigkeit in einem vorgegebenen Zeitintervall dem Blut zugeführt wird.

7. Vorrichtung nach einem der Ansprüche 1-6, **dadurch gekennzeichnet, dass** die Mittel zum Bestimmen des Phasenwinkels mindestens einer Oberschwingung des oszillierenden Drucksignals eine Fourier-Analyse-Einrichtung (32a) aufweisen, die zur Ermittlung des Phasenwinkels eine Fourier-Analyse des oszillierenden Drucksignals durchführt.

8. Vorrichtung nach einem der Ansprüche 1-7, **dadurch gekennzeichnet, dass** die Mittel zum Generieren des oszillierenden Drucksignals eine in dem arteriellen Zweig (20) des extrakorporalen Blutkreislaufs (9) angeordnete Blutpumpe (6), insbesondere eine okkludierende Pumpe, aufweisen.

9. Vorrichtung nach einem der Ansprüche 1-8, **dadurch gekennzeichnet, dass** die Mittel zum Messen des oszillierenden Drucksignals einen im venösen Zweig (21) des extrakorporalen Blutkreislaufs (9) angeordneten Drucksensor (31) aufweisen.

**Claims**

1. A device for the detection of disruptions of the blood flow in an extracorporeal blood circuit during an extracorporeal blood treatment with an extracorporeal blood treatment apparatus, which has an extracorporeal blood circuit (9) with an arterial branch (20) leading to a blood treatment unit (1) and a venous branch (21) leading away from the blood treatment unit, wherein the device for the detection of disruptions of the blood flow comprises:

means (6) for generating an oscillating pressure signal propagated in the extracorporeal blood circuit,
means (31) for measuring the oscillating pressure signal and means (32) for analysing the oscillating pressure signal,

**characterised in that**

the means (32) for analysing the oscillating pressure signal comprise means (32a) for determining the phase angle of at least one harmonic of the oscillating pressure signal and means (32b) for detecting a change in the phase angle.

2. The device according to claim 1, **characterised in that** the means (32b) for detecting a change in the phase angle are designed in such a way that the change in the phase angle of at least one harmonic is ascertained in a preset time interval and the temporal change in the phase angle of the harmonic is compared with a preset limiting value, wherein a disruption is detected if the amount of the change in the phase angle is greater than the preset limiting value.

3. The device according to claim 1 or 2, **characterised in that** the device for the detection of disruptions of the blood flow also comprises means (25, 32) for undertaking an intervention into the control of the extracorporeal blood treatment apparatus.

4. The device according to any one of claims 1-3, **characterised in that** the extracorporeal blood treatment apparatus is a haemodialysis and/or haemofiltration apparatus and the blood treatment unit (1) is a dialyser or a filter, wherein the extracorporeal blood treatment apparatus comprises:

an ultrafiltration device (18), which withdraws fluid at a preset ultrafiltration rate from the blood flowing through the extracorporeal blood circuit, and a substitution device (19), which supplies substitution fluid to the blood flowing through the extracorporeal blood circuit upstream and/or downstream of the dialyser (1) or filter.

5. The device according to claim 4, **characterised in that** the means (25, 32) for undertaking an intervention into the control of the haemodialysis and/or haemofiltration apparatus controls the ultrafiltration device (18) when a disruption is detected, in such a way that the ultrafiltration rate is reduced in a preset time interval.

6. The device according to claim 4, **characterised in that** the means (25, 32) for undertaking an intervention into the control of the haemodialysis and/or haemofiltration apparatus controls the substitution device (19) when a disruption is detected, in such a way that a specific quantity of substitution fluid is fed to the blood in a preset time interval upstream of the dialyser (1) or filter.

7. The device according to any one of claims 1-6, **characterised in that** the means for determining the phase angle of at least one harmonic of the oscillating pressure signal comprises a Fourier analysis device (32a), which carries out a Fourier analysis of the oscillating pressure signal in order to ascertain the phase angle.

8. The device according to any one of claims 1-7, **characterised in that** the means for generating the oscillating pressure signal comprise a blood pump (6), in particular an occluding pump, arranged in the arterial branch (20) of the extracorporeal blood circuit (9).

9. The device according to any one of claims 1-8, **characterised in that** the means for measuring the oscillating pressure signal comprise a pressure sensor (31) arranged in the venous branch (21) of the extracorporeal blood circuit (9).

**Revendications**

1. Dispositif de détection de troubles du débit sanguin dans une circulation sanguine extracorporelle pendant un traitement extracorporel du sang à l'aide d'un dispositif de traitement extracorporel du sang comprenant une circulation sanguine extracorporelle (9) dotée d'une branche artérielle (20) menant à une unité de traitement du sang (1) et d'une branche veineuse (21) partant de l'unité de traitement du sang, le dispositif de détection des troubles du débit sanguin comprenant :

◆ des moyens (6) destinés à générer un signal de pression oscillant se propageant dans la circulation sanguine extracorporelle,

EP 1 687 045 B1

- des moyens (31) destinés à mesurer le signal de pression oscillant et

- des moyens (32) destinés à analyser le signal de pression oscillant, **caractérisé en ce que**

  - les moyens (32) destinés à analyser le signal de pression oscillant comprennent des moyens (32a) destinés à déterminer l'angle de phase d'au moins une oscillation harmonique du signal de pression oscillant et des moyens (32b) destinés à détecter une modification de l'angle de phase.

2. Dispositif selon la revendication 1, **caractérisé en ce que** les moyens (32b) destinés à détecter une modification de l'angle de phase sont conçus de telle sorte que la modification de l'angle de phase d'au moins une oscillation harmonique dans un intervalle de temps prédéterminé soit déterminée et que la modification temporelle de l'angle de phase de l'oscillation harmonique soit comparée avec une valeur seuil prédéterminée, un trouble étant détecté lorsque la valeur de la modification de l'angle de phase est supérieure à la valeur seuil prédéterminée.

3. Dispositif selon la revendication 1 ou 2, **caractérisé en ce que** le dispositif de détection des troubles du débit sanguin comprend en outre des moyens (25, 32) destinés à intervenir dans la commande du dispositif extracorporel de traitement du sang.

4. Dispositif selon l'une quelconque des revendications 1 à 3, **caractérisé en ce que** le dispositif extracorporel de traitement du sang est un dispositif d'hémodialyse et/ou d'hémofiltration et l'unité de traitement du sang (1) est un dialyseur ou un filtre, le dispositif de traitement extracorporel du sang comprenant :

   - un appareil d'ultrafiltration (18) prélevant le liquide du sang circulant à travers la circulation sanguine extracorporelle à une vitesse d'ultrafiltration prédéterminée et un appareil de substitution (19) amenant le liquide de substitution au sang circulant à travers la circulation sanguine extracorporelle en amont et/ou en aval du dialyseur (1) ou filtre.

5. Dispositif selon la revendication 4, **caractérisé en ce que** les moyens (25, 32) destinés à intervenir dans la commande du dispositif d'hémodialyse et/ou d'hémofiltration commandent l'appareil d'ultrafiltration (18) lors de la détection d'un trouble de telle sorte que la vitesse d'ultrafiltration soit réduite dans un intervalle de temps prédéterminé.

6. Dispositif selon la revendication 4, **caractérisé en ce que** les moyens (25, 32) destinés à intervenir dans la commande du dispositif d'hémodialyse et/ou d'hémofiltration commandent l'appareil de substitution (19) lors de la détection d'un trouble, de telle sorte qu'une quantité déterminée de liquide de substitution soit amenée au sang dans un intervalle de temps prédéterminé en amont du dialyseur (1) ou filtre.

7. Dispositif selon l'une quelconque des revendications 1 à 6, **caractérisé en ce que** les moyens destinés à déterminer l'angle de phase d'au moins une oscillation harmonique du signal de pression oscillant comprennent un appareil d'analyse de Fourier (32a) réalisant une analyse de Fourier du signal de pression oscillant pour déterminer l'angle de phase.

8. Dispositif selon l'une quelconque des revendications 1 à 7, **caractérisé en ce que** les moyens destinés à générer le signal de pression oscillant comprennent une pompe à sang (6) disposée dans la branche artérielle (20) de la circulation sanguine extracorporelle (9), en particulier une pompe à occlusion.

9. Dispositif selon l'une quelconque des revendications 1 à 8, **caractérisé en ce que** les moyens destinés à mesurer le signal de pression oscillant comprennent un capteur de pression (31) disposé dans la branche veineuse (21) de la circulation sanguine extracorporelle (9).

Fig. 1

EP 1 687 045 B1

**Fig.2**

Fig.3

**EP 1 687 045 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1175917 A1
- DE 3806248 A1
- US 20020174721 A1